(19) Europäisches Patentamt
European Patent Office
Office européen des brevets

(11) Veröffentlichungsnummer: **0 017 000**
**B1**

(12) # EUROPÄISCHE PATENTSCHRIFT

(45) Veröffentlichungstag der Patentschrift: **16.12.81**

(51) Int. Cl.³: **B 01 J 37/02,** B 01 J 23/88, C 07 C 51/235 // B01J35/08, B01J35/10

(21) Anmeldenummer: **80101151.1**

(22) Anmeldetag: **07.03.80**

(54) **Verfahren zur Herstellung von Schalenkatalysatoren und ihre Verwendung.**

(30) Priorität: **12.03.79 DE 2909671**

(43) Veröffentlichungstag der Anmeldung: **15.10.80 Patentblatt 80/21**

(45) Bekanntmachung des Hinweises auf die Patenterteilung: **16.12.81 Patentblatt 81/50**

(84) Benannte Vertragsstaaten: **BE DE FR**

(56) Entgegenhaltungen:
**DE-A-1 936 233**
**DE-A-2 456 113**
**DE-A-2 608 546**
**DE-C-867 811**
**US-A-3 956 377**
**US-A-4 077 912**

(73) Patentinhaber: **BASF Aktiengesellschaft**, ***Carl-Bosch-Strasse 38, D-6700 Ludwigshafen (DE)***

(72) Erfinder: **Krabetz, Richard, Dr., Unterer Waldweg 8, D-6719 Kirchheim (DE)**
Erfinder: **Fermann, Walter, Dr., Pommernstrasse 105, D-6800 Mannheim 1 (DE)**
Erfinder: **Engelbach, Heinz, Dr., Kropsburgstrasse 24, D-6703 Limburgerhof (DE)**
Erfinder: **Palm, Peter, Hauptstrasse 46, D-6711 Gerolsheim (DE)**
Erfinder: **Sommer, Karl Dr., Bozener Strasse 19, D-6700 Ludwigshafen (DE)**
Erfinder: **Spahn, Heinrich, Dr., Molkestrasse 13, D-6800 Mannheim 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 017 000 B1

# Verfahren zur Herstellung von Schalenkatalysatoren und ihre Verwendung

Die Erfindung betrifft ein neues Verfahren zur Herstellung von Schalenkatalysatoren durch Aufbringen von pulverförmigem katalytisch aktivem Material in Gegenwart von Wasser auf bewegte Trägerteilchen.

Aus der GB-PS 1 346 943 ist es bekannt, daß man Schalenkatalysatoren, bestehend aus einem Träger und einer darauf aufgebrachten Schale aus katalytisch aktivem Material dadurch herstellen kann, daß man das katalytisch aktive Material mit Hilfe des Plasmaspritz- oder Flammspritzverfahrens auf den Träger aufbringt. Voraussetzung für die Anwendung des Verfahrens ist die Schmelzbarkeit mindestens einer Hauptkomponente bei der Arbeitstemperatur des Flammspritz- oder des Plasmabrenners. Die auf bekannte Weise des Auftrags mittels Zerstäubung in einem Vergleichsbeispiel erhaltenen Katalysatoren mit $V_2O_5$, $MoO_3$ und/oder $WO_3$ enthaltenden Schalen liefern zwar bei der Oxidation von Indanen im Vergleich zu den Ausbeuten des Verfahrens der GB-PS 1 346 943 (45%) nur eine Ausbeute von 13,8%, doch läßt die Aktivität von über die Schmelze hergestellten aktiven Massen für viele Verfahren sehr zu wünschen übrig, und die für das Verfahren erforderliche Herstellung von gut rieselfähigem Material ist schwierig und aufwendig.

Aus der US-PS 3 956 377 ist ein spezielles Verfahren zur Herstellung von Schalenkatalysatoren für die Gasphasenoxidation von (Meth)Acrolein zu (Meth)Acrylsäure bekannt, bei dem man z. B. Molybdänoxid, Vanadinoxid und Wolframmetallpulver durch Erhitzen unter Rückfluß in Wasser löst, die erhaltene Aufschlämmung eindampft und mehrere Tage bei 115°C trocknet. Die auf diese Weise erhaltene aktive Katalysatormasse wird dann auf das zuvor mit Wasser angefeuchtete Trägermaterial aufgebracht, indem das feuchte Trägermaterial in einem Pulver des aktiven Katalysators gewälzt wird. Ein derartiges Verfahren ist auch aus der US-PS 4 077 912 bekannt. Katalysatoren, die auf diese Weise hergestellt sind, sind häufig wenig aktiv, insbesondere da sie hinsichtlich des Anteils an katalytisch aktiver Masse in der Schale begrenzt sind durch die Porosität des Trägers und damit sein Wasseraufnahmevermögen. Ein weiterer Nachteil der Methode besteht darin, daß der Befeuchtungsgrad der jeweiligen Oberflächenschicht während des Beschichtungsvorgangs keinen stationären Wert besitzt, was die Haftfestigkeit beeinträchtigt und zu Verklebungen der Trägerteilchen unter Bildung von Zwillingen und Drillingen und zu unterschiedlichen Schalenstärken führen kann.

Aus der US-PS 2 035 606 (Seite 3), der GB-PS 1 385 496 und der DE-OS 2 626 887 ist es schließlich bekannt, daß man Schalenkatalysatoren herstellen kann, indem man wäßrige Suspension des katalytisch aktiven Materials auf die bewegten Trägerteilchen sprüht, wobei die Trägerteilchen z. B. gemäß der US-PS 3 562 185 und der GB-PS 1 385 496 auf Temperaturen über 150°C, bei Mitverwendung von Kunstharzdispersionen auf etwa 70 bis 130°C erhitzt sein können. Dabei erhält man jedoch bei der schlagartigen Verdampfung des Suspensionsmediums, wenn die Trägerteilchen Temperaturen von z. B. 150°C und mehr aufweisen, im allgemeinen Schalenkatalysatoren mit unzureichender Abriebfestigkeit der katalytisch aktiven Schale und hohe Verluste an aktivem Material bei der Beschichtung. Bei Mitverwendung von Kunstharz-Dispersionen wird der Beschichtungsvorgang durch schwer kontrollierbare Filmbildungsprozesse erschwert, außerdem muß das Kunstharz anschließend herausgebrannt werden, wobei eine Lockerung des Strukturgefüges und eine thermische Schädigung der Aktivität eintreten kann. Bei dem Verfahren der DE-OS 2 626 887, bei dem bei Temperaturen von 25 bis 80°C gearbeitet werden kann, kann es zu Verklebungen der besprühten Teilchen kommen, und die Haftfestigkeit der Schale ist häufig unbefriedigend.

Es wurde nun gefunden, daß man Schalenkatalysatoren mit

a) einem inerten Träger eines Teilchendurchmessers von mindestens 100 µm und einer Oberfläche bis 20 m²/g und
b) einer auf der äußeren Oberfläche und in der oberflächennahen Randzone der Trägerteilchen festhaftenden Schale, die das katalytisch aktive Material enthält,

durch Aufbringen des katalytisch aktiven Materials in Form eines Pulvers einer Teilchengröße von höchstens 300 µm in Gegenwart von Wasser auf den stark bewegten Träger mit Vorteil herstellen kann, indem man auf die gegebenenfalls mit bis 95% ihres Wasseraufnahmewertes vorbefeuchteten Trägerteilchen kontinuierlich mit jeweils konstanter Dosiergeschwindigkeit räumlich getrennt voneinander 1 bis 40 g/Minute/Liter Träger an katalytisch aktivem Material und Wasser mit einem Gewichtsverhältnis von katalytisch aktivem Material zu Wasser von 1 : 1 bis 30 : 1 derart aufbringt, daß der Wassergehalt der sich bildenden Schale kleiner ist als der maximale Sättigungsgrad der Schale des katalytisch aktiven Materials.

Werden bei dem neuen Verfahren poröse Träger eingesetzt, so soll deren mittlerer Porendurchmesser bevorzugt über 20 µm betragen, und die Teilchengröße des katalytisch aktiven Materials soll dann kleiner sein als der mittlere Porendurchmesser des Trägers.

Es zeigte sich überraschend, daß das erfindungsgemäße Verfahren im Gegensatz zu den bekannten Verfahren auch bei der Herstellung von Katalysatorchargen im technischen Maßstab Schalenkatalysatoren mit sehr einheitlicher Verteilung der aktiven Masse auf den Trägerteilchen ohne Agglomeration der Katalysatorteilchen, einheitlichem Schüttgewicht und mit einer hohen Abriebfestigkeit liefert, was eine besonders gleichmäßige und rasche Füllung von Rohrbündelreaktoren ermöglicht und zu einer Verbesserung der durchschnittlichen Selektivität und Raumzeitausbeute technischer Chargen beiträgt. Ein weiterer überraschender Vorteil des erfindungsgemäßen Verfahrens besteht darin, daß auch bei Gewichtsanteilen der aktiven Masse von über 100 und sogar 150 Gew.-%, bezogen auf den Träger, bzw. bei Schalendicken über 0,8 mm, z. B. 1,5 mm, noch Schalenkatalysatoren mit guter Abriebfestigkeit erhalten werden, was beim technischen Einsatz von Schalenkatalysatoren, z. B. bei der Oxidation von Isobuten zu Methacrolein, von Bedeutung ist.

Für das neue Verfahren zur Herstellung von Schalenkatalysatoren kommen die üblichen Trägermaterialien, wie Aluminiumoxide, z. B. $\alpha$-$Al_2O_3$, Siliziumdioxid, Thoriumdioxid, Zirkondioxid und Silikate, beispielsweise Magnesiumsilikat und Aluminiumsilikat sowie Siliziumcarbid in Frage. Die Teilchen des Trägermaterials können ungeformt oder geformt sein, wobei geformte Träger mit deutlich ausgebildeter Oberflächenrauhigkeit, beispielsweise Kugeln oder Ringe, bevorzugt werden. Der Teilchendurchmesser der Träger soll mindestens 100 $\mu$m betragen und liegt vorzugsweise im Bereich von 0,5 bis 12 mm, insbesondere von 1 bis 9 mm. Die Träger können porös oder unporös sein, doch sollen poröse Träger einen mittleren Porendurchmesser von mindestens 20 $\mu$m, vorzugsweise mindestens 50 $\mu$m, aufweisen. Bei Verwendung poröser Träger soll die Teilchengröße des aktiven Materials im allgemeinen kleiner sein als der mittlere Porendurchmesser des Trägers. Sollen die fertigen Katalysatoren für Reaktionen eingesetzt werden, bei denen bei Temperaturen oberhalb etwa 350°C gearbeitet wird, so werden Träger bevorzugt, deren innere Oberfläche unter 5, insbesondere unter 2 $m^2/g$ und deren Porosität im allgemeinen 0 bis 10% beträgt und vorzugsweise kleiner als 5% ist. Sollen die fertigen Schalenkatalysatoren bei Temperaturen eingesetzt werden, die unterhalb etwa 300°C liegen, so können auch Träger mit Porositäten über 5 oder über 10% vorteilhaft sein. Träger, die eine Porosität unter 30%, insbesondere unter 5% aufweisen, kommen für die Herstellung der neuen Schalenkatalysatoren bevorzugt in Betracht.

Die Art bzw. die Zusammensetzung des katalytisch aktiven Materials ist für das neue Verfahren nicht spezifisch; grundsätzlich kann nach dem neuen Verfahren praktisch jedes katalytisch aktive Material auf Träger aufgebracht werden. In Frage kommen alle bekannten üblichen, insbesondere für stark exotherme Reaktionen geeignete Katalysatormassen, vor allem auf Basis von Metalloxiden, Metalloxidmischungen und/oder Metallmischoxiden, wie sie insbesondere für katalytische Oxidationsreaktionen, beispielsweise die katalytische Oxidation von $\alpha$-Olefinen, wie Propen und Isobutylen, zu $\alpha,\beta$-olefinisch ungesättigten Aldehyden, wie besonders Acrolein und Methacrolein sowie von Kohlenwasserstoffen, wie Butan oder Buten zu Maleinsäureanhydrid, o-Xylol zu Phthalsäureanhydrid, o- und p-substituierte Alkylbenzole zu o- und p-substituierten Benzaldehyden, die oxidative Dehydrierung von Kohlenwasserstoffen oder Alkoholen sowie die Oxidation von Aldehyden eingesetzt werden. Derartige katalytisch aktive Massen sind beispielsweise in der GB-PS 1 491 750, GB-PS 1 249 290, GB-PS 1 351 218, GB-PS 1 416 099 und der GB-PS 1 305 810 beschrieben. Von besonderem Interesse sind die für die (Meth)Acroleinoxidation zu (Meth)Acrylsäure üblichen katalytisch aktiven Massen auf Basis von Oxiden bzw. Mischoxiden, die Molybdän, Vanadin, Wolfram, Kupfer oder/und Mangan oder/und Eisen sowie gegebenenfalls untergeordneten Mengen an Alkali- und Erdalkalimetallen, die gegebenenfalls weitere metalloxidische Komponenten enthalten und wie sie besonders in der DE-OS 2 626 887 beschrieben sind. Bewährt haben sich insbesondere für die Gasphasenoxidation von Acrolein mit Sauerstoff enthaltenden Gasen zur Acrylsäure unter an sich üblichen Druck- und Temperaturbedingungen Schalenkatalysatoren, die nach dem neuen Verfahren hergestellt sind und deren katalytisch aktives Material die Zusammensetzung

$$Mo_{12}V_aW_bMe_c^1Me_d^2Me_e^3O_x$$

hat, bei der

| | |
|---|---|
| $Me^1$ | für Cu und/oder Fe und gegebenenfalls zusätzlich Mn, |
| $Me^2$ | für mindestens ein Metall aus der Gruppe Sb, Sn, Cr, Nb, Ta und Ni, |
| $Me^3$ | für ein Alkali und/oder Erdalkalimetall |

stehen und bei der
a = 0,5 bis 15, vorzugsweise 2 bis 6, b = 0,1 bis 6, vorzugsweise 0,5 bis 3, c = 0,1 bis 6, vorzugsweise 1 bis 3, d = 0 bis 6, vorzugsweise 0 bis 1 und e = 0 bis 0,1, insbesondere 0,001 bis 0,1 bedeuten und x für die Zahl der Atome steht, die zur Absättigung der Valenzen der anderen Bestandteile erforderlich ist.

Für die Methacroleinoxidation sind aktive Massen auf der Grundlage von Molybdän, Vanadin, Phosphor und Alkali oder $NH_4$ enthaltenden Mischoxiden besonders geeignet.

Bei dem neuen Verfahren wird das katalytisch aktive Material in der Regel in seiner fertigen katalytisch aktiven Form, beispielsweise als calciniertes Mischoxid, das nach der Calcinierung auf die vorgesehene Teilchengröße vermahlen ist, aufgebracht. In manchen Fällen ist es aber auch von Vorteil, das Calcinieren des katalytisch aktiven Materials erst nach der Herstellung des Schalenkatalysators vorzunehmen. Die Teilchengröße des aktiven katalytischen Materials liegt unter 300, meist unter

150 µm, vorzugsweise zu 90 Gew.-% unter 50 µm, insbesondere zwischen 1 und 45 µm. Sollen die Katalysatoren als aktive katalytische Masse Metalle enthalten, so können diese gegebenenfalls als Oxide aufgebracht werden, wobei dann die erhaltenen Schalenkatalysatoren nach dem Trocknen reduziert werden, was unmittelbar vor ihrem Einsatz im technischen Reaktor durchgeführt werden kann. Bevorzugt wird jedoch auch in diesem Fall eine Beschichtung des Trägers mit der aktiven katalytischen Masse, d. h. hier z. B., mit den katalytisch aktiven Metallen, die auf oxidische Komponenten aufgefällt sein können, so daß eine Nachbehandlung nicht erforderlich ist.

Die Menge des aktiven katalytischen Materials auf dem fertigen Schalenkatalysator beträgt im allgemeinen 3 bis 300, insbesondere 5 bis 200 Gew.-%, bezogen auf das Trägergewicht.

Bei der praktischen Durchführung des Verfahrens kann in drehbaren Behältern, z. B. geneigten Drehtellern, die vorzugsweise einen Neigungswinkel von 30 bis 60° aufweisen, oder mit Vorteil in Dragierkesseln gearbeitet werden. Die Behälter sind vorzugsweise zu 20 bis 80% ihres Volumens mit den Trägerteilchen gefüllt, und ihre Umdrehungszahl beträgt im allgemeinen je nach Durchmesser 1 bis 60 je Minute, wodurch die Trägerteilchen stark bewegt werden. Das Wasser kann z. B. durch eine Ein- oder Zweistoffdüse auf die bewegten Trägerteilchen gesprüht werden. Bei Verwendung einer Zweistoffdüse muß gegebenenfalls das in das Treibgas verdampfende Wasser bei der Menge des aufzusprühenden Wassers berücksichtigt werden.

Das trockene katalytisch aktive Material kann z. B. mittels einer Dosierschnecke oder Bandwaage mit Förderrinne und gegebenenfalls Verteilereinrichtung auf die Trägerteilchen aufgebracht werden. Die zeitlich zugeführte Menge an trockenem katalytisch aktivem Material beträgt 1 bis 40 g, vorzugsweise 1 bis 25 g je Liter Trägerteilchen je Minute, und das Gewichtsverhältnis von trockenem katalytisch aktivem Material zu Wasser liegt zwischen 1 : 1 und 30 : 1 und beträgt vorzugsweise von 2 : 1 bis 10 : 1, wobei die Dosiergeschwindigkeiten beider Komponenten praktisch konstant gehalten und so gewählt werden, daß der Wassergehalt der sich bildenden Schale kleiner ist als der maximale Sättigungsgrad der Schale des katalytisch aktiven Materials und im allgemeinen von 40 bis 97, insbesondere von 40 bis 92, vorzugsweise von 60 bis 87% des maximalen Sättigungsgrades beträgt. Der maximale Sättigungsgrad der Schale entspricht einem Feuchtigkeitsgehalt, bei dem die Katalysatorteilchen zu verkleben beginnen und nicht mehr als isolierte Teilchen durch den Apparat rollen. Der maximale Sättigungsgrad der Schale des katalytisch aktiven Materials ist näherungsweise gegeben durch die Menge Wasser in g, die 100 g des katalytisch aktiven Materials in der Form seines Einsatzes bei erfindungsgemäßer Herstellung der Schalenkatalysatoren bei Raumtemperatur (22° C) aufnimmt, wenn man das Pulver in einer Porzellanschale rührt und das Wasser innerhalb von 5 bis 10 Minuten zutropft, bis alles Pulver agglomeriert ist und die Oberfläche der Agglomerate feucht und klebrig zu werden beginnt.

Die zunehmend beschichteten Trägerteilchen sollen während der Beschichtung in der Randschicht also ausreichend feucht sein, um das kontinuierlich zugeführte Pulver vollständig aufzunehmen und fest zu binden, jedoch nicht so feucht, daß die Trägerteilchen in der Wassersprühzone agglomerieren und nicht mehr als isolierte Teilchen rollen.

Bei dem erfindungsgemäßen Verfahren sollen die Trägerteilchen vor Zugabe des Katalysatorpulvers meist mit mindestens 0,1% ihres Gewichtes an Wasser vorbefeuchtet werden. Dabei ist die erforderliche Vorbefeuchtung von der Wasseraufnahme der Trägerteilchen abhängig und nimmt mit deren Porosität zu. Unporöse Trägerteilchen brauchen nicht vorbefeuchtet zu werden.

Bei Trägerteilchen einer geringen Porosität bis 5%, wie sie für das neue Verfahren vorgezogen werden, hat man mit einer Vorbefeuchtung von 0,1 bis 2%, vorzugsweise 0,5 bis 1,5%, des Gewichtes der Trägerteilchen gute Erfahrungen gemacht, wobei jedoch maximal mit 95% des Wasseraufnahmewertes gearbeitet wird. Bei Porositäten über 5% sind im allgemeinen Vorbefeuchtungen mit 5 bis 30, vorzugsweise 10 bis 25% des Wasseraufnahmewertes günstig. Trägerteilchen mit einer Porosität bis 5%, d. h. möglichst unporöse Träger, sollen möglichst rauhe Oberflächen haben und ergeben Schalenkatalysatoren, die im allgemeinen bei vergleichbarer Aktivität selektiver sind als vergleichbare Schalenkatalysatoren, zu deren Herstellung wesentlich porösere Trägerteilchen verwendet sind.

Der Wasseraufnahmewert ist die Menge Wasser in g, die von 100 g des eingesetzten Trägermaterials aufgenommen werden, wenn man das Trägermaterial durch Aufkochen mit Wasser sättigt, durch Eintauchen in kaltes Wasser auf Zimmertemperatur abkühlt und dann das oberflächlich anhaftende Wasser in einem schwach befeuchteten Papierfilter abtropfen läßt.

Von Bedeutung für die Ausbildung einer haftfesten, gleichmäßig dicken Schale ist auch die relative Lage der Katalysatorpulver- und Wasserzudosierung, die räumlich getrennt voneinander erfolgen soll. Insbesondere soll die Einführung des Katalysatorpulvers im allgemeinen außerhalb des Zerstäubungskegels des vorzugsweise durch eine Düse eingesprühten Wassers erfolgen, um eine vorzeitige Agglomeration der Katalysatorpulverteilchen zu unterbinden. Die in der Wasser-Sprühzone in der Oberflächenrandschicht auf einen zeitlich konstanten unvollständigen Befeuchtungsgrad gehaltenen, teilweise beschichteten Trägerteilchen nehmen mit konstanter Geschwindigkeit das

zugeführte Katalysatorpulver auf, das sich durch die — z. B. rollende — Bewegung der Katalysatorteilchen auf der äußeren Teilchenoberfläche zu einer zusammenhängenden Schale verdichtet. Besonders bei hohem Katalysatorpulver : Wasser-Verhältnis und langsamer Zugabe von Katalysatorpulver und Wasser wird schon während des Beschichtungsvorgangs eine harte, abriebfeste Schale erhalten. Im allgemeinen ist es vorteilhaft, nach beendeter Beschichtung den beschichteten Träger bei Temperaturen von 60 bis 150°C, insbesondere von 70 bis 120°C, nachzutrocknen. Die Trocknung kann mit Vorteil im Beschichtungsapparat durch indirekte Beheizung oder bevorzugt durch Einleiten von Luft, z. B. einer Temperatur von 20 bis 300, insbesondere von 20 bis 150°C, unter langsamer Bewegung der Schüttung erfolgen, so lange, bis entweder die Temperatur der Schüttung einen um 100°C liegenden Wert erreicht hat oder bis eine bestimmte Restfeuchte der Schalenkatalysatorteilchen erreicht ist. Die Restfeuchte soll im allgemeinen weniger als 2%, vorzugsweise 0,1 bis 1,5 Gew.-%, bezogen auf den Schalenkatalysator, betragen.

Die beschichteten Katalysatoren können, falls erforderlich, durch eine Nachbehandlung, z. B. Calcinierung oder Reduktion, in den aktiven Zustand überführt werden, z. B., wenn die Bestandteile des katalytisch aktiven Materials in inaktiver Form auf den Träger aufgebracht wurden. Bei der bevorzugten Ausführungsform ist eine Nachbehandlung nicht erforderlich, da hier das aktive Material im katalytisch aktiven calcinierten Zustand auf die Trägerteilchen aufgebracht ist.

Ein besonderer Vorzug des neuen Verfahrens besteht darin, daß in einem Arbeitsgang Schalenkatalysatoren mit schichtförmig aus zwei oder mehr unterschiedlichen aktiven Massen aufgebauten Schalen hergestellt werden können.

Die in den folgenden Beispielen angegebenen Prozente sind Gewichtsprozente.

## Beispiel 1

### a) Herstellung des Katalysators

Es wird eine katalytisch aktive Masse der Zusammensetzung $Mo_{12}V_3W_{1,2}Cu_{2,2}O_{49,3}$ wie folgt hergestellt:

Eine Lösung von 32,3 kg Ammoniumparawolframat, 35 kg Ammoniummetavanadat und 212 kg Ammoniumheptamolybdat in 1400 kg Wasser wird bei 95°C mit einer Lösung von 26 kg Kupfer(II)-acetat in 350 kg Wasser sowie 10 kg Kupferhydroxidcarbonat versetzt und bei 110°C sprühgetrocknet. Das Produkt wird anschließend mit 0,15 kg Wasser je kg Festprodukt verknetet, bei 140°C getrocknet und 2¹/₂ Stunden bei von 230 bis 350°C stufenweise ansteigender Temperatur einer ersten Calcinierung, anschließend 2¹/₂ Stunden bei 400°C einer zweiten Calcinierung unterworfen. Das calcinierte, katalytisch aktive Material wird auf eine Teilchengröße von kleiner 80 µm gemahlen. Der maximale Sättigungsgrad des Pulvers beträgt 25%.

80 l (112 kg), als Katalysator-Träger handelsübliche oberflächenrauhe unporöse Magnesiumsilikatkugeln (Rosenthal Fl/Sp) eines Durchmessers von 6 mm werden in einem Dargierkessel von 200 l Inhalt nach Vorbefeuchtung mit 1,1 kg Wasser mit 4,1 kg Wasser bei einer Dosiergeschwindigkeit von 1,281 g $H_2O$/min je l Träger und einer Drehzahl des Dragierkessels von 8 Umdrehungen/min kontinuierlich durch eine Düse besprüht. Gleichzeitig wurden 24 kg des Katalysatorpulvers mit einer Geschwindigkeit von 7,5 g Pulver/l Träger je min über eine Bandwaage und Schüttelrinne außerhalb des Sprühkegels der Wasser-Zerstäubungsdüse kontinuierlich zudosiert. Hierbei beträgt der Wassergehalt in der aufwachsenden Schalenschicht im Mittel 87% des maximalen Sättigungsgrades. Während der Beschichtung wird das zugeführte Pulver vollständig aufgenommen, eine Agglomeration der Katalysatorteilchen ist nicht zu beobachten. Nach beendeter Beschichtung wird der Katalysator mit 110°C heißer Luft bis auf einen Wasser-Restgehalt von 0,5 Gew.-% getrocknet.

#### Abriebtest

1,16 l Katalysator wird über eine Bandwaage in ein Testrohr von 25 mm innerem Durchmesser und 3 m Länge mit konstanter Geschwindigkeit innerhalb von 30 sec eingefüllt, anschließend wieder aus dem Rohr abgelassen und die Abriebmenge bestimmt. Sie betrug weniger als 0,01%.

### b) Gasphasenoxidation von Acrolein enthaltenden Reaktionsgasen der Propylenoxidation

In ein Reaktionsrohr eines freien Durchmessers von 25 mm wird 1 l des Schalenkatalysators eingefüllt und das Reaktionsrohr in einem Salzbad auf 278°C geheizt. Man leitet dann stündlich 2217 Normalliter eines Gasgemisches über den Katalysator, das 4 Volumenprozent Acrolein, 0,1 Volumenprozent Acrylsäure, 5,15 Volumenprozent Wasserdampf, 5,6 Volumenprozent Sauerstoff, 85 Volumenprozent Stickstoff und 0,15 Volumenprozent Nebenprodukte der Propylenoxidation, Essigsäure, Formaldehyd, Maleinsäure, CO und $CO_2$ enthält. Der Umsatz des Acroleins beträgt 98 Mol-%, die Ausbeute an Acrylsäure 95 Mol-%.

Beispiel 2

a) Herstellung des Katalysators

Es wird eine katalytisch aktive Masse der molaren Zusammensetzung $1\ V_2O_5 \cdot 0{,}026\ K_2O$ hergestellt, indem man Kaliumcarbonat und Vanadiumpentoxid als Pulver mischt, auf 670° C erhitzt und danach die flüssige Schmelze in einer Blechwanne erstarren läßt. Die erstarrte Masse wird anschließend gebrochen und zu einem Pulver mit einer Teilchengröße kleiner 50 µm gemahlen. Der maximale Sättigungsgrad des Pulvers beträgt 21,2%.

120 g Pulver werden bei einer Dosiergeschwindigkeit von 20 g Pulver/min auf 1000 g $\alpha$-$Al_2O_3$-Kugeln (Wasseraufnahmewert 1%) von 8 mm Durchmesser, die nach Vorbefeuchtung mit 6 g $H_2O$ kontinuierlich, aber räumlich getrennt von der Pulverzugabe mit 18 g Wasser bei einer Sprühgeschwindigkeit von 3 g/min besprüht werden, aufgranuliert. Die Drehzahl des verwendeten 30-cm-Drehtellers beträgt 35 Umdrehungen/min. Der Wassergehalt der aufwachsenden Schale beträgt im Mittel 94% des maximalen Sättigungsgrades. Der erhaltene Schalenkatalysator weist eine extrem hohe Abriebfestigkeit auf, nachdem er bei 100° C auf einen Rest-Feuchtigkeitsgehalt von 0,2% getrocknet wurde. Nach dem in Beispiel 1 beschriebenen Falltest betrug der Abrieb weniger als 0,01 Gew.-%.

b) Verwendung des Katalysators für die Oxidation von p-t-Butyltoluol zu p-t-Butylbenzaldehyd (TBBA) und p-t-Butylbenzoesäure (TBBS)

Über eine 20-cm³-Probe des Katalysators wird in einem röhrenförmigen Laborreaktor eines freien Durchmessers von 25 mm ein Gemisch aus 2 Nl/Stunde p-tert-Butyltoluol, 20 Nl/Stunde Luft und 15 Nl/Stunde Wasserdampf bei 412°C geleitet. Es wird ein Umsatz von 22 Mol-% und Ausbeuten an TBBA von 6,9 Mol-% und an TBBS von 7,1 Mol-% erzielt. Die Gesamtselektivität für beide Produkte beträgt damit 68 Mol-%.

Beispiel 3

a) Herstellung des Katalysators

Es wurde ein katalytisch aktives Material der Zusammensetzung

$$Mo_{12}Bi_1Fe_3Ni_1Co_7B_2Sb_{0,1}K_{0,14}O_{56,7}$$

nach der in der GB-PS 1 491 750, Beispiel 1, gegebenen Vorschrift hergestellt und calciniert.

Die erhaltene Masse wird auf eine Teilchengröße kleiner 30 µm gemahlen. Ihr maximaler Sättigungsgrad beträgt 33,4%. 156 g des Katalysatorpulvers werden wie folgt unter Zusatz von 62,2 g Wasser auf 100 g (= 76 ml) unporöse Steatitkugeln von 3 mm Durchmesser aufgebracht:

In einem Drehteller von 30 cm Durchmesser werden die mit 1,0 g Wasser (1% ihres Gewichtes) vorbefeuchteten Steatitkugeln kontinuierlich mit 17,3 g Katalysatorpulver/l Träger/min mit einer Dosierschnecke beschickt und mit 6,9 g Wasser/l Träger/min durch eine Zweistoffdüse unter Verwendung von 12,5 Nl/min Luft von Raumtemperatur als Treibgas bei 35 Umdrehungen je Minute und einer Neigung des Tellers von 45° besprüht, wobei das Katalysatorpulver außerhalb des Wassersprühkegels auf die rollenden Kugeln auftrifft. Dabei beträgt der Wassergehalt der aufwachsenden Schale etwa 67% des maximalen Sättigungsgrades. Anschließend wird der Katalysator bei 110° C bis auf einen Wassergehalt von 0,3% getrocknet. Der mittlere Durchmesser der erhaltenen Schalenkatalysatorteilchen beträgt 4,7 mm, entsprechend einer mittleren Schichtdicke der Schale von 0,85 mm. Die Abriebfestigkeit des Katalysators ist sehr gut. Nach dem in Beispiel 1 beschriebenen Falltest mit einer 50-g-Probe betrug der Abrieb < 0,01 Gew.-%.

b) Verwendung des Katalysators für die Oxidation von Isobuten zu Methacrolein

43 cm³ des Katalysators werden in einem röhrenförmigen Laborreaktor (Rohrdurchmesser: 15 mm) stündlich mit einem Gasgemisch aus 3 Nl Isobuten, 37,2 Nl Luft und 24 Nl Wasserdampf bei einer Badtemperatur von 376°C beschickt. Dabei werden 94 Mol-% des Isobutens umgesetzt. Die Ausbeute an Methacrolein beträgt 80 Mol-% und die Ausbeute an Methacrylsäure 1 Mol-%, die Gesamtselektivität für beide Produkte 87 Mol-%.

Beispiel 4

a) Herstellung des Katalysators

Es wird eine katalytisch aktive Masse der Zusammensetzung

$$Mo_{12}Ni_{8,5}Fe_2Bi_1P_{0,06}Na_{0,18}K_{0,06}Si_{10}O_x$$

wie folgt hergestellt:

561 g wäßrige Wismutnitratlösung (11% Wismut, 5% freie Salpetersäure), 1117 g wäßrige Nickelnitratlösung (13,2% Nickel), 239 g wäßriges Eisennitrat in 1240 g Wasser werden in dieser Reihenfolge zusammengegeben. Zu dieser Lösung wird eine Lösung aus 625 g Ammoniumheptamolybdat, 2,3 g 75%iger Phosphorsäure und 452 g 25%ige Ammoniaklösung in 3320 g Wasser gegeben. Schließlich werden noch 354 g 50%iges Kieselsol zugegeben und die entstandene Suspension sprühgetrocknet.

500 g des Sprühgutes werden unter Zusatz von

62 g Wasser und 0,34 g 48%iger Kalilauge 1 1/2 Stunden geknetet, zu 4,5-mm-Strängen verformt und 24 Stunden bei 120° C getrocknet.

Die getrockneten Stränge werden 2 Stunden bei 360° C unter Luftzutritt calciniert und anschließend auf eine Teilchengröße kleiner 300 µm gemahlen. (Der Natriumgehalt der Masse stammt aus natürlichen Verunreinigungen der Komponenten.)

Der maximale Sättigungsgrad beträgt 48%. 2600 g des Pulvers werden mit einer Dosiergeschwindigkeit von 17,4 g/l/Träger/min kontinuierlich auf 2500 g (=1,78 l) der mit insgesamt 850 g Wasser und mit einer Geschwindigkeit von 5,69 g/l/Träger/min kontinuierlich besprühten Trägerkugeln aufgranuliert. Die als Träger verwendeten Magnesiumsilikatkugeln mit einer Wasseraufnahme von 1% und einem Durchmesser von 1,5 bis 2,5 mm werden vor der Beschichtung mit 32 g Wasser vorbefeuchtet und während der Beschichtung in einem Drehteller von 50 cm Durchmesser bei einer Drehzahl von 14 U/min unter einem Neigungswinkel von 45° gerollt. Der mittlere Befeuchtungsgrad beträgt 71% des maximalen Sättigungsgrades des Katalysatorpulvers. Nach beendeter Beschichtung wird der Katalysator 16 Stunden bei 80° C getrocknet und anschließend 1 1/2 Stunden bei 600° C im Luftstrom calciniert. Die Abriebfestigkeit des Katalysators ist sehr gut; nach dem in Beispiel 1 beschriebenen Falltest in einem Rohr mit 21 mm Durchmesser beträgt der Abrieb nur 0,025%.

Während der Beschichtung ist wie in den Beispielen 1 bis 3 keine Agglomeration der Katalysatorteilchen zu beobachten.

b) Verwendung des Katalysators für die Oxidation von Propylen zu Acrolein

800 cm³ des Schalenkatalysators werden in ein Stahlrohr von 3,6 m Länge und einem Innendurchmesser von 21 mm eingefüllt und durch das das Rohr umgebende Salzbad auf 342 bis 343° C geheizt. Über den Katalysator wird stündlich ein Gemisch aus 80 Nl Propylen, 800 Nl Luft und 800 Nl Stickstoff geleitet. Der Propylenumsatz beträgt 95,2 Mol-% und die Ausbeute an Acrolein + Acrylsäure 89,5 Mol-%. Die Selektivität der Acrolein + Acrylsäure-Bildung ergibt sich zu 94%.

Bei einer Badtemperatur von 332° C erhält man einen Umsatz von 88,7 Mol-%, eine Ausbeute von Acrolein und Acrylsäure von 84,6 Mol-% und eine Selektivität von 95,4 Mol-%.

Beispiel 5

350 g der nach Beispiel 1 hergestellten aktiven Masse wird auf einen porösen, kugelförmigen $\alpha$-$Al_2O_3$-Träger von 4,7 mm Durchmesser, einer Porosität von 30% bzw. einer Wasseraufnahme von 17 Gew.-% wie folgt aufgebracht:

0,92 l (1000 g) Träger werden in einer Dragiertrommel von 30 cm Durchmesser mit 36 g Wasser (21,2% des Wasseraufnahmewertes) vorbefeuchtet. Anschließend werden 350 g aktive Masse mit einer Dosiergeschwindigkeit von 11,7 g/min/l Träger auf den gleichzeitig mit insgesamt 36 g Wasser kontinuierlich besprühten Träger aufgebracht, wobei die Dragiertrommel mit 34 UpM gedreht wird. Nach beendeter Beschichtung wird der Katalysator bei 120° C bis auf einen Wassergehalt von 0,4% getrocknet.

Zur Prüfung der Abriebfestigkeit werden 50 g des Katalysators in einem Drehteller von 30 cm Durchmesser 3 Minuten bei einer Umdrehungszahl des Tellers von 35 UpM gerollt und der Abrieb bestimmt. Er beträgt weniger als 0,05%, bezogen auf die Einwaage.

Vergleichsversuch A

Beispiel 5 wird wiederholt, wobei jedoch der Träger mit 72 g Wasser, d. h. 42,3% seines Wasseraufnahmewertes, mit Wasser vorbefeuchtet, kein Wasser gleichzeitig mit dem Pulver aufgesprüht und das Pulver in 5 gleichen Portionen zugegeben wird. Nur 340 g des Pulvers bleiben am Träger haften, wobei zudem die Masseverteilung unbefriedigend ist. Nach der Trocknung bleiben 13,5 Gew.-% des Katalysators zu Zwillings- und Drillingsteilen verklebt oder sind nur unzureichend beschichtet. Die Abriebfestigkeit des Katalysators ist sehr schlecht: Die Abriebsmenge in dem in Beispiel 5 angegebenen Test beträgt 4,7%, bezogen auf das Gesamtgewicht.

Vergleichsversuch B

Beispiel 5 wird wiederholt mit der Maßgabe, daß der Träger nicht vorbefeuchtet wird. Man erhält einen Katalysator, dessen Abriebfestigkeit wesentlich schlechter ist als die des in Beispiel 5 erhaltenen Katalysators, der Abrieb beträgt über 3%.

Beispiele 6 bis 10

Nach Beispiel 1 werden weitere Schalenkatalysatoren mit aktiven Massen der herkömmlichen Zusammensetzungen

(6) $Mo_{12}W_{1,2}V_3Cu_{2,2}Fe_{0,2}O_x$;
(7) $Mo_{12}W_{1,2}V_3Cu_{2,0}Mn_{0,4}O_x$;
(8) $Mo_{12}W_{2,4}V_{4,6}Cu_{2,2}Cr_{0,6}O_x$;
(9) $Mo_{12}W_{1,2}V_3Cu_2Sn_{0,5}O_x$;
(10) $Mo_{12}W_{1,2}V_3Cu_{2,2}Nb_{0,2}Cr_{0,6}O_x$

hergestellt, wobei die Zusatzkomponenten als Eisen(III)acetat, Manganacetathydrat, Chromnitrat, Zinn(II)oxid, Nioboxid eingesetzt werden. Der Abrieb in dem Abriebtest (vgl. Beispiel 1) beträgt jeweils weniger als 0,02 Gew.-%. Die

Katalysatoren sind für die Gasphasen-Oxidation von Acrolein zu Acrylsäure unter sonst üblichen Bedingungen sehr gut geeignet.

Beispiel 11

Gemäß Beispiel 1 der DE-AS 2 122 664 wird ein pulverförmiges Gemisch von 24,06 g $V_2O_5$ und 0,44 g $H_6TeO_6$ bei 670° C geschmolzen. Nach dem Erkalten wird die Masse gebrochen und zu einem Pulver einer Teilchengröße $< 50\ \mu$ gemahlen.

Die so hergestellte katalytisch aktive Masse wird gemäß Beispiel 2 auf 340 g Mullitkugeln eines Durchmessers von 5 bis 7 mm unter kontinuierlicher Befeuchtung der Kugeln mit insgesamt 4,5 g Wasser aufgranuliert.

Die beschichteten Kugeln werden auf 0,4% Restfeuchte getrocknet.

Der Abriebverlust des fertigen Katalysators nach der bei Beispiel 1 angegebenen Methode beträgt weniger als 0,08 Gew.-%, bezogen auf die aktive Masse.

Der Katalysator eignet sich z. B. bei etwa 430° C sehr gut für die Gasphasenoxidation von 1-Methyl-3-phenylindan zu Anthrachinon.

**Patentansprüche**

1. Verfahren zur Herstellung von Schalenkatalysatoren mit
a) einem inerten Träger eines Teilchendurchmessers von mindestens 100 µm und einer Oberfläche bis 20 $m^2/g$ und
b) einer auf der äußeren Oberfläche und in der oberflächennahen Randzone der Trägerteilchen festhaftenden Schale, die das katalytisch aktive Material enthält,

durch Aufbringen des katalytisch aktiven Materials in Form eines Pulvers einer Teilchengröße von höchstens 300 µm in Gegenwart von Wasser auf den stark bewegten Träger, dadurch gekennzeichnet, daß man auf die gegebenenfalls mit bis 95% ihres Wasseraufnahmewertes mit Wasser vorbefeuchteten Trägerteilchen kontinuierlich mit jeweils konstanter Dosiergeschwindigkeit räumlich getrennt voneinander 1 bis 40 g/Minute/Liter Träger an katalytisch aktiven Material und Wasser mit einem Gewichtsverhältnis von katalytisch aktivem Material zu Wasser von 1 : 1 bis 30 : 1 derart aufbringt, daß der Wassergehalt der sich bildenden Schale kleiner ist als der maximale Sättigungsgrad der Schale des katalytisch aktiven Materials.

2. Verfahren nach Anspruch 1, dadurch gekennzeichnet, daß die Trägerteilchen eine Porosität von weniger als 5% aufweisen und mit 0,1 bis 2% ihres Gewichtes mit Wasser vorbefeuchtet werden.

3. Verfahren nach Anspruch 1, dadurch gekennzeichnet, daß die Trägerteilchen eine Porosität von über 5% aufweisen und mit 5 bis 30% ihres Wasseraufnahmewertes vorbefeuchtet werden.

4. Verfahren nach Anspruch 1 bis 3, dadurch gekennzeichnet, daß die Teilchengröße des katalytisch aktiven Materials kleiner als 50 µm ist.

5. Verfahren nach Anspruch 1 bis 4, dadurch gekennzeichnet, daß der Schalenkatalysator auf einen Wassergehalt von weniger als 2 Gew.-% getrocknet wird.

6. Verfahren zur Herstellung eines Schalenkatalysators nach den Ansprüchen 1 bis 5, dadurch gekennzeichnet, daß das katalytisch aktive Material die Zusammensetzung

$$Mo_{12}V_aW_bMe_c^1Me_d^2Me_e^3O_x$$

hat, in der

- $Me^1$ für Cu und/oder Fe sowie gegebenenfalls zusätzlich Mn,
- $Me^2$ für mindestens ein Metall aus der Gruppe Sb, Sn, Cr, Nb, Ta und Ni,
- $Me^3$ für ein Alkali und/oder Erdalkalimetall

stehen und
a = 0,5 bis 15, b = 0,1 bis 6, c = 0,1 bis 6, d = 0 bis 6 und e = 0 bis 0,1
bedeuten und x für die Zahl der Atome steht, die zur Absättigung der Valenzen der anderen Bestandteile erforderlich ist.

7. Verwendung von Schalenkatalysatoren, die nach Anspruch 6 hergestellt sind, für die Oxidation von Acrolein mit Sauerstoff enthaltenden Gasen zu Acrylsäure in der Gasphase.

**Claims**

1. A process for the preparation of a coated catalyst comprising

a) an inert carrier having a particle diameter of not less than 100 µm and a surface area of up to 20 $m^2/g$ and
b) a coating which firmly adheres to the outer surface and in the edge zone, near the surface, of the carrier particles, and which contains the catalytically active material,

by applying the catalytically active material, in the form of a powder of a particle size of not more than 300 µm, in the presence of water, to the vigorously agitated carrier, characterized in that catalytically active material in an amount of from 1 to 40 g/minute/liter of carrier, and water in a weight ratio of catalytically active material to water of from 1 : 1 to 30 : 1, are applied continuously and spatially separate from one another, each at a constant speed, onto the carrier particles, which may or may not have been pre-moistened with water in an amount of up to 95% of the particle absorbency, in such a way that the water content of the coating which forms is less than the maximum degree of saturation of the coating of catalytically active material.

2. A process as claimed in claim 1, characterized in that the carrier particles have a porosity of less than 5% and are pre-moistened with from 0.1 to 2% of water, based on the weight of carrier particles.

3. A process as claimed in claim 1, characterized in that the carrier particles have a porosity of more than 5% and are pre-moistened with an amount of water corresponding to from 5 to 30% of their water absorbency.

4. A process as claimed in claims 1 to 3, characterized in that the particle size of the catalytically active material is less than 50 µm.

5. A process as claimed in claims 1 to 4, characterized in that the coated catalyst is dried to a water content of less than 2% by weight.

6. A process for the preparation of a coated catalyst as claimed in claims 1 to 5, characterized in that the catalytically active material has the composition

$$Mo_{12}V_aW_bMe_c^1Me_d^2Me_e^3O_x$$

where

| | |
|---|---|
| $Me^1$ | is Cu and/or Fe, with or without Mn, |
| $Me^2$ | is at least one metal from the group consisting of Sb, Sn, Cr, Nb, Ta and Ni, |
| $Me^3$ | is an alkali metal and/or alkaline earth metal and |

a is from 0.5 to 15, b is from 0.1 to 6, c is from 0.1 to 6, d is from 0 to 6 and e is from 0 to 0.1, and x is the number of atoms required to saturate the valencies of the other constituents.

7. Use of a coated catalyst prepared as claimed in claim 6 for the gas phase oxidation of acrolein to acrylic acid by means of an oxygen-containing gas.

**Revendications**

1. Procédé de préparation de catalyseurs à particules enrobées, constitués de:

a) un support inerte en particules d'un diamètre d'au moins 100 µm et avec une surface active pouvant atteindre 20 m²/g;
b) un enrobage adhérant fortement à la surface extérieure et à la zone marginale proche de la surface des particules du support, qui contient la matière à activité catalytique,

par dépôt de la matière à activité catalytique sous forme d'une poudre d'une granulométrie maximale de 300 µm en présence d'eau sur le support maintenu en turbulence, caractérisé en ce que l'on dépose sur les particules du support, éventuellement pré-mouillées par de l'eau jusqu'à 95% de leur capacité d'absorption de l'eau, en continu, en deux courants séparés dans l'espace, avec des débits dosés constants, 1 à 40 g par mn et par l de support de matière à activité catalytique et de l'eau dans des proportions pondérales matière à activité catalytique-eau dans un rapport de 1 : 1 à 30 : 1, de telle façon que la teneur en eau de l'enrobage qui se forme est inférieure au degré de saturation maximale de la matière à activité catalytique.

2. Procédé suivant la revendication 1, caractérisé en ce que les particules du support possèdent une porosité inférieure à 5% et sont pré-mouillées par de l'eau à raison de 0,1 à 2% de leur poids.

3. Procédé suivant la revendication 1, caractérisé en ce que les particules du support possèdent une porosité supérieure à 5% et sont pré-mouillées par de l'eau jus-qu'à 5 à 30% de leur capacité d'absorption de l'eau.

4. Procédé suivant les revendications 1 à 3, caractérisé en ce que les particules de la matière à activité catalytique possèdent une granulométrie inférieure à 50 µm.

5. Procédé suivant les revendications 1 à 4, caractérisé en ce que le catalyseur à particules enrobées est séché jusqu'à une teneur résiduelle en eau inférieure à 2% en poids.

6. Procédé de préparation d'un catalyseur à particules enrobées suivant les revendications 1 à 5, caractérisé en ce que la matière à activité catalytique possède la composition

$$Mo_{12}V_aW_bMe_c^1Me_d^2Me_e^3O_x$$

dans laquelle

| | |
|---|---|
| $Me^1$ | représente Cu et (ou) Fe et éventuellement aussi Mn, |
| $Me^2$ | désigne au moins un métal du groupe Sb, Sn, Cr, Nb, Ta et Ni, |
| $Me^3$ | désigne un métal alcalin et (ou) alcalino-terreux, |

a = 0,5 à 15; b = 0,1 à 6; c = 0,1 à 6; d = 0 à 6 et e = 0 à 0,1 et x correspond au nombre d'atomes nécessaires pour saturer les valences des autres ingrédients.

7. Utilisation de catalyseurs à particules enrobées, préparés par le procédé suivant la revendication 6, pour l'oxydation en phase gazeuse de l'acroléine par des gaz contenant de l'oxygène en acide acrylique.